# EUROPEAN PATENT SPECIFICATION

(11) **EP 2 654 730 B1**
(45) Date of publication and mention of the grant of the patent: **23.11.2016**
(21) Application number: 11807693.4
(22) Date of filing: 23.12.2011
(51) Int. Cl.: A61K 9/20, A61K 31/343

(54) **HIGH DRUG LOAD PHARMACEUTICAL FORMULATIONS COMPRISING DRONEDARONE AND ITS PHARMACEUTICALLY ACCEPTABLE SALTS**
PHARMAZEUTISCHE FORMULIERUNGEN MIT HOHEM WIRKSTOFFGEHALT MIT DRONEDARON UND PHARMAZEUTISCH AKZEPTABLEM SALZ DARAUS
FORMULATIONS PHARMACEUTIQUES À CHARGE MÉDICAMENTEUSE ÉLEVÉE COMPRENANT DE LA DRONÉDARONE ET DES SELS PHARMACEUTIQUEMENT ACCEPTABLES DE CELLES-CI

(30) Priority: 24.12.2010 SI 201000452
(43) Date of publication of application: 30.10.2013
(73) Proprietor: KRKA, d.d., Novo mesto, 8501 Novo mesto (SI)
(72) Inventor: ZAKELJ, Mojca Segula, 2000 Maribor (SI); BENKIC, Primoz, 1000 Ljubljana (SI); PRESKAR, Maja, 8270 Krsko (SI); KROSELJ, Vesna, 8000 Novo mesto (SI); VRECER, Franc, 8351 Straza (SI); OSOLNIK, Renata, 8351 Straza (SI)
(74) Representative: Hoffmann Eitle
(86) International application number: PCT/EP2011/073991
(87) International publication number: WO 2012/085284

(56) References cited:
- WO-A2-2011/135581
- WO-A2-2011/135582
- CN-A- 101 152 154

## Description

### Technical Field

The subject of the invention includes a high drug load pharmaceutical composition comprising dronedarone or its pharmaceutically acceptable salts, in particular dronedarone HCl as the active pharmaceutical ingredient, comprising micronized particles of the active ingredient and optionally ionic surfactants.

### Background of the Invention

Dronedarone with its chemical name N-(2-butyl-3-(p-(3-(dibutylamino)propoxy)benzoyl)-5-benzofuranyl)methanesulfonamide and chemical formula C₃₁H₄₄N₂O₅S was first developed by Sanofi-Synthelabo and used for the treatment of cardiac arrhythmia. The solubility of dronedarone HCl in aqueous media is very low and pH dependent. The solubility of dronedarone hydrochloride is maximal at pH 3-5, around 1-2mg/ml, and it decreases a lot at pH around 6-7. The solubility at pH=7 is only 10µg/ml. Due to the poor solubility of dronedarone hydrochloride the bioavailability of solid oral dosage forms is low. Tablets comprising dronedarone hydrochloride are on the market under the trade name Multaq^{®}.

WO 98/58643 discloses a solid pharmaceutical composition in tablet form comprising dronedarone or a pharmaceutically acceptable salt thereof and a nonionic hydrophilic surfactant which allegedly prohibits the active ingredient from precipitation at pH values 6-7 and makes it possible to maintain the solubilisation of active substance in neutral medium and therefore increases the bioavailability of dronedarone HCl by which the food effect on the bioavailability of dronedarone hydrochloride is said to be reduced.

US20040044070 describes an injection of dranedarane HCl where a *β* -cyclodextrin derivative is added to the buffering system (pH 3-5) and therefore the solubility of the active ingredient is said to be increased.

CN 100560067 discloses low drug load solid medicinal compositions comprising dronedarone hydrochloride and 1-10% sodium lauryl sulfate wherein the drug load is at the maximum 40 wt.%. The drawback of such a formulation is the size of the final dosage form which decreases the patient compliance.

CN 102078307A discloses a pharmaceutical composition of dronedarone hydrochloride in the form of dispersible tablets wherein dronedarone hydrochloride is micronized by a solid dispersion technology and wherein the bioavailability of active substance is allegedly improved by using polyethylene glycole 4000 as a surfactant. WO 2011/113320 claims to solve the problem of solubility and bioavailability of dronedarone hydrochloride by preparing pharmaceutical composition comprising mixture of pharmaceutically acceptable one or more amphiphilic lipid surfactants and one or more phospholipids.

CN101152154 discloses the use of micronization of dronedarone salts for use in oral compositions.

WO 2011/135581 discloses the pharmaceutical composition of dronedarone or salts thereof wherein the surfactant, particularly nonionic hydrophilic surfactant(s), is omitted. The inventors assert that by judicial selection of excipients in its optimum concentrations the dronedarone formulations can be made with improved release profile and bioavailability.

WO 2011/135582 discloses the pharmaceutical composition of dronedarone or salts thereof comprising one or more surfactants other than nonionic hydrophilic surfactants. The application claims that this composition exhibits better or at least same bioavailability as compared to the tablets marked under the trade name Multaq^{®}. However, this teaching has no support in the description of the application.

To increase the oral bioavailability of an active compound with relatively poor water solubility, various concepts have been described in the past. Thus, solutions of active compounds are frequently used which can be filled, for example, into soft gelatine capsules. This option is not applicable, however, in the present case, since in the necessary dose strength, capsule sizes would result which are no longer swallowable.

There is thus a need in the art for high drug load solid oral dosage forms comprising dronedarone or its pharmaceutically acceptable salts, in particular dronedarone hydrochloride, with an improved dissolution profile.

### Summary of the Invention

It has now surprisingly been found that the solubility of dronedarone or its pharmaceutically acceptable salt, in particular dronedarone hydrochloride, can also be sufficiently increased by micronization of the active ingredient, optionally in combination with ionic surfactants, and/or the hydrophilization of the active ingredients particles in the solid oral dosage form.

Accordingly, the present invention provides a tablet with high drug loading comprising a pharmacologically effective amount of dronedarone or its pharmaceutical acceptable salt, preferably dronedarone hydrochloride, present in an amount of from about 40% to 90%, and micronized, or co-micronized with a filler, as defined by claim 1. In other embodiments the dronedarone is preferably present in an amount of from about 50% to 90 %, most preferably in an amount of from about 60% to 90 % by weight based on the total weight of the tablet and the hydrochloride salt.

Tablets according to the invention surprisingly provide for the administration of a pharmacologically effective amount of dronedarone or its pharmaceutically acceptable salt, preferably dronedarone hydrochloride in a smaller size than was hitherto possible for a given unit dose of a pharmacologically effective amount of dronedarone or its pharmaceutically acceptable salt, preferably dronedarone hydrochloride in micronized form and optionally comprising a ionic surfactant. The tablets of the invention are, despite the high drug loading, small, and, therefore, convenient to administer. This leads to a better patient compliance.

### Brief Description of Figures

Figure 1: Results of dissolution tests with dronedarone HCl in phosphate buffer pH 4.5.
Figure 2: Results of dissolution tests with dronedarone HCl in phosphate buffer pH 6.7.

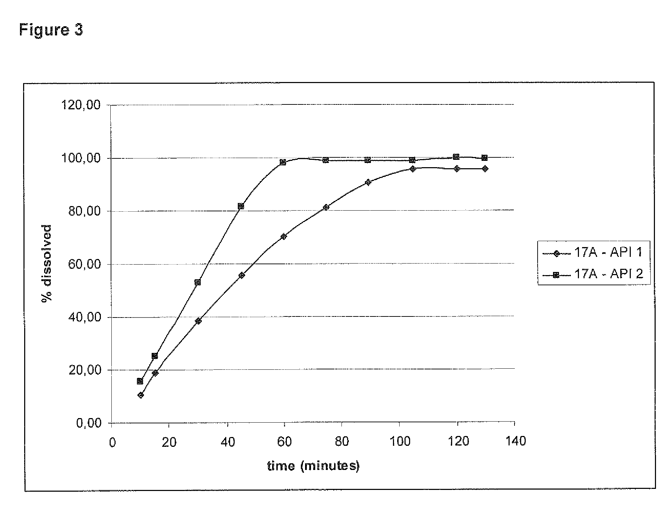

Figure 3: Comparison of dissolution rate of the same pharmaceutical compositions comprising different active substances.

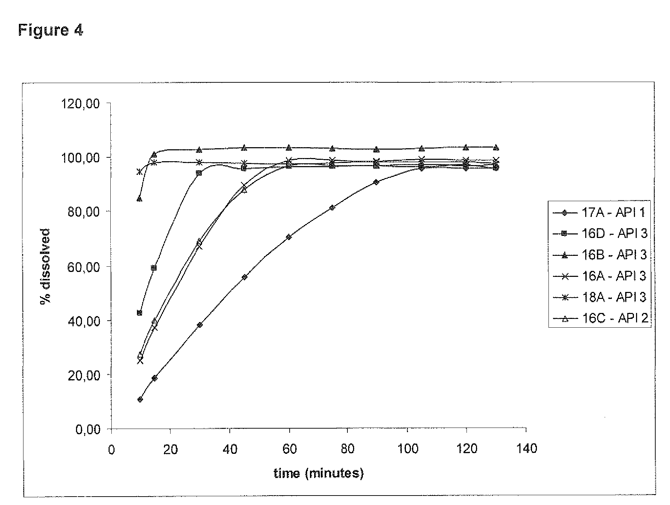

Figure 4: Comparison of dissolution rate of different pharmaceutical compositions comprising different active substances.

### Detained Description of the Invention

There are three commercially important processes for making compressed tablets: wet/moist granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets.

The properties of the drug, its dosage forms and the economics of the operation will determine the selection of the best process for tabletting. Generally, both wet granulation and direct compression are used in developing a tablet.

The dry granulation method may be used where one of the constituents, either the drug or the diluent, has insufficient cohesive properties to be tabletted. The method consists of blending, slugging the ingredients, dry screening, lubrication and compression.

The wet granulation method is used to convert a powder mixture into granules having suitable flow and cohesive properties for tableting. The procedure consists of mixing the powders in a suitable blender followed by addition of the granulating solution under shear to the mixed powders to obtain a granulation. The damp mass is then screened through a suitable screen and dried by tray drying or fluidized bed drying. Alternatively, the wet mass may be dried and passed through a mill. The overall process includes weighing, dry powder blending, wet granulating, drying, milling, blending lubrication and compression.

According to the present invention a process of preparing a granulation liquid may comprise the following steps:
i) suspending the active ingredient in a pharmaceutically acceptable solvent
ii) optionally addition of further excipients
iii) homogenizing and/or micronization of the suspension

In another embodiment according to the present invention a process of preparing a granulation liquid may comprise dissolving or suspending the binder in a pharmaceutically acceptable solvent.

In another embodiment according to the present invention a pharmaceutically acceptable solvent alone may be used as a granulation liquid in the granulation process.

In general, powders do not have sufficient adhesive or cohesive properties to form hard, strong granules. A binder is usually required to bond the powder particles together due to the poor cohesive properties of most powders. Heat and moisture sensitive drugs cannot usually be manufactured using wet granulation. The large number of processing steps and processing time are problems due to high level manufacturing costs. Wet granulation has also been known to reduce the compressibility of some pharmaceutical excipients, such as microcrystalline cellulose.

An alternative means of granulation is also thermoplastic granulation. Thermoplastic granulation may be accomplished by means of a process by which granules are obtained through the addition of either a molten binder or a solid binder which melts during the process.

Direct compression is regarded as a relatively quick process where the powdered materials are compressed directly without changing the physical and chemical properties of the drug. The active ingredient(s), direct compression excipients and other auxiliary substances, such as a glidant and lubricant are blended in a twin shell blender or a similar low shear apparatus before being compressed into tablets. This type of mixing was believed to be essential in order to prepare "pharmaceutically acceptable" dosage forms. Some pharmaceutical scientists believe that the manner in which a lubricant is added to a formulation must be carefully controlled. Accordingly, lubricants are usually added to a granulation by gentle mixing. It is also believed that prolonged blending of a lubricant with a granulation can materially affect hardness and disintegration time for the resulting tablets.

It is an object of the present invention to address the above-mentioned need in the art by providing a pharmaceutical composition and dosage form for orally administering dronedarone or its pharmaceutically acceptable salts, particularly dronedarone hydrochloride.

Accordingly, the present invention provides a tablet with high drug loading comprising a pharmacologically effective amount of dronedarone or its pharmaceutically acceptable salt, preferably dronedarone hydrochloride, present in an amount of from about 40% to 90%, preferably in an amount of from about 50% to 90 %, most preferably in an amount of from about 60 % to 90 % by weight based on the total weight of the tablet and the hydrochloride salt.

In the first embodiment of the present invention provides a high drug load composition and dosage form comprising a therapeutically effective amount of dronedarone or its pharmaceutically acceptable salt, preferably dronedarone hydrochloride in micronized form.

The inventors of the present invention surprisingly found out the use of micronized dronedarone or its pharmaceutically acceptable salts greatly contributes towards an improved dissolution rate of the active substance. It is thus highly advantageous that active substance having particle size and/or specific surface area within the specific ranges is used.

According to a first variant of the first embodiment the particle size of the active ingredient is d₉₀<20 µm, preferably d₉₀<15 µm, more preferably d₉₀ < 6 micrometers and/ or its specific surface area is preferably more than 2,5 m²/g, most preferably more than 5 m²/g. The specific surface area can be determined using the BET method. According to another variant of the first embodiment d₉₀ of the micronized form is between 6 µm and 70 µm, preferably between 6 and 30 µm and more preferably between 6 and 10 µm of the active ingredient, preferably of dronedarone hydrochloride.

As used herein d₉₀<x means that at least 90% by volume of the particles have a particle size below x. The particle size can be determined by laser light scattering for instance using a Malvern Mastersizer Apparatus MS 2000 equipped with a Hydro S dispersion unit. Silicon fluid F10 may be used as dispersion medium.

Overmicronized active ingredient, preferably dronedarone hydrochloride, according to the present invention means the active substance having d₉₀ less than 6 µm and having specific surface area more than 8 m²/g.

According to one variant of this embodiment of the invention, micronized dronedarone hydrochloride is used, wherein the active substance has d₉₀ of more than 6 µm and a specific surface area less than 8 m²/g.

For the manufacturing of the solid compositions according to the present invention also specially designed particles of the active compound can be used such as spherically crystallized, porous particles where at least 30% of the particle surface area is internal.

The micronized active ingredient to be used according to the first embodiment of the invention may be obtained by means of any micronisation process of reducing primary or crystalline particle size by any method known from the state of the art such as for example dry or wet mills such as for example air jet and/or ball milling, high shear mill or high pressure homogenizer or dry mills such as cutting mills, pin/cage mills, hammer mills, jet mills, fluidized bed jet mills and ball mills or Hosokawa Alpine agitated ball mill, ultrasound processors, or wet mills such as Hielscher ultrasonic processor, and in particular high shear mixers and colloid mills, such as Turrax and colloid mills produced by Fryma.

The possible chargeability and secondary agglomeration due to increased surface energy of the particles can be very problematic during the micronization and further processing. This problem can be avoided by using wet micronization or co-milling and/or co-micronization of the active ingredient with at least one excipient. This excipient can be selected from the group of soluble saccharides such as lactose, sucrose, mannitol, sorbitol etc.; colloidal silica or natural silicates having high specific surface area, preferably of more than 50m²/g; water soluble polymers, disintegrants and/or ionic surfactants. Co-processing can be performed by dry or wet milling or micronization processes.

The micronized active ingredient and at least one pharmaceutically acceptable excipient can be optionally processed to obtain a granulate by dry or liquid granulation processes such as slugging or roller compaction as dry processes or wet or melt granulation as liquid granulation processes or can be processed in another embodiment of the present invention into spherical granules such as pellets.

In a second embodiment of the invention, the subject of the invention includes a high drug load pharmaceutical composition with increased solubility comprising dronedarone or its pharmaceutically acceptable salts, in particular dronedarone HCl in micronized form as the active pharmaceutical ingredient and optionally comprising ionic surfactants. The active ingredient may further be hydrophilized by the technological process of preparing the oral solid dosage form.

In the second embodiment the invention describes the use of ionic surfactants. Ionic surfactants used may be chosen from the group of anionic, cationic, or ampholytic surfactants or any combinations thereof. The cationic surfactants may be selected from the group consisting of organic quaternary ammonium halides, R₄N⁺Cl⁻, cetrimide, a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula [C₆H₅CH₂N⁺(CH₃)₂R]Cl⁻, where R represents a mixture of alkyls from C₈H₁₇ to C₁₈H₃₇., benzethonium chloride, bronidox, cetrimonium bromide, cetrimonium chloride, dimethyldioctadecylammonium chloride, lauryl methyl gluceth-10 hydroxypropyl dimonium chloride, tetramethylammonium hydroxide. The anionic surfactant may be selected from the group consisting of organic sulfonates (RSO₃⁻) like e.g. sodium docusate (dioctyl sodium sulfosuccinate) or bis(2-ethylhexyl) sulfosuccinate sodium salt or sulfates (ROSO₃⁻), potassium laurate(CH₃(CH₂)₁₀COO⁻K⁺) and sodium lauryl sulfate (CH₃(CH₂)₁₁SO₄⁻Na⁺), ammonium lauryl sulfate, dioctyl sodium sulfosuccinate, MBAS assay, perfluorobutane sulfonic acid, perfluorononanoic acid, perfluorooctane sulfonic acid, perfluorooctanoic acid, potassium lauryl sulfate, soap, soap substitute, sodium dodecyl sulfate, sodium dodecylbenzene sulfonate, sodium stearate. The most preferred anionic surfactant is sodium lauryl sulfate or sodium docusate. The ampholytic surfactants may be selected from suffobetaines, RN⁺(CH₃)₂CH₂CH₂SO₃⁻), N-dodecyl-N,N-dimethylbetaine, lecithines such as for example natural lecithins, phospholipids, phosphatidylcholine, hydrogenated phosphatidylcholine, lysolecithin, preferably Phospholipon^{®} 90 G and Phospholipon^{®} 90 H and C₁₂H₂₅N⁺(CH₃)₂CH₂COO⁻, amphiphile, CHAMPS detergent, cocamidopropyl betaine, cocamidopropyl hydroxysultaine, hydroxysultaine, sodium lauroamphoacetate. Some surfactants, e.g. sodium docusate, may also have the function of a disintegrant.

According to another variant of this second embodiment any combinations of ionic surfactants may be used as a surfactant.

According to another embodiment 2a polyethylene glycol glycerides of mono-, di- and triglycerides and mono-, and diesters of polyethylene glycol may be used as a surfactant in combination with the micronized active ingredient as described in the above first embodiment. Preferably Gelucire® may be used as a surfactant in accordance with this embodiment. The HLB value of the surfactant of this embodiment is not particularly limited. For instance, any Gelucire® as well as Gefucire® mixtures having different HLB values may be used.

According to yet another embodiment 2b any combination of ionic surfactants with polyethylene glycol glycerides of mono-, di- and triglycerides and mono-, and diesters of polyethylene glycol may be used as a surfactant in combination with the micronized active ingredient as described in the above first embodiment. The ionic embodiments to be used according to this embodiment 2b may be selected from the ionic embodiments described above in relation to the second embodiment. The polyethylene glycol glycerides of mono-, di- and triglycerides and mono-, and diesters of polyethylene glycol may be as described in embodiment 2a above.

According to still another embodiment 2c the pharmaceutical composition according to the present invention as described in the above first embodiment is free of surfactant.

For the preparation of solid compositions in tablet form or hard gelatin or hydroxypropylmethyl cellulose capsule, from 1% to 20% by weight of surfactant calculated on the weight of active ingredient in base form, preferably from 1% to 15%, more preferably 1% to 5 %, will be used, for example. As a non-limiting guide, the amount of active ingredient can range from 50 to 500 mg per administration unit in tablet form calculated to the dose of free base, which entails the incorporation of an amount of surfactant of between 0.5 and 100 mg, preferably from 0.5 and 75 mg, more preferably 0.5 and 25 mg. These amounts of surfactant are safe and acceptable with pharmaceutical forms such as tablets or gelatin capsule.

In a preferred manner, solid pharmaceutical compositions of the invention, for example in tablet or capsule form, can contain from 200 to 400 mg of the active ingredient calculated in the form of base and from 5% to 15%, more particularly 10%, by weight of ionic hydrophilic surfactant relative to the active ingredient in base form. For packaging in the form of powder in a unit sachet, from 1% to 50% by weight of ionic hydrophilic surfactant relative to the active ingredient in base form may be used. The compositions according to the invention can further comprise other pharmaceutical excipients generally used in the development of oral pharmaceutical forms. Any of the three processes for making compressed tablets mentioned above may be applied.

Dry granulation according to present invention can be performed by mixing micronized active ingredient and at least one ionic surfactant with optional additional excipient selected from binders, disintegrants, glidants, lubricants and/or diluents or mixture thereof and dry granulating the mixture. The obtained material is crushed or milled and optionally sieved to obtained granulate with desired particle size and particle size distribution.

The obtained granulate according to processes of present invention can be processed into tablets by mixing obtained granulate with additional excipients selected from disintegrant, binder, diluents, glidant, lubricant or mixture thereof or filled directly or upon mixing it with at least one additional excipient into capsules or sachets. The particle size of the granulate is d₅₀ < 400 micrometers, preferably d₅₀ < 300 micrometers and/or d₉₀ < 500 micrometers, preferably d₉₀ < 400 micrometers.

The term "pharmaceutical acceptable excipient" as used herein refers to additives useful for converting pharmacologically active compounds into pharmaceutical dosage forms which are suitable for administration to patients. Suitable excipients include fillers, binders, disintegrants, surfactants, lubricants, glidants and coloring agents. Other pharmaceutically acceptable excipients can also be included.

The composition according to the invention typically comprises at least one filler. Suitable fillers include monosaccharides and oligosaccharides such as glucose, fructose, saccharose, lactose (anhydrous and monohydrate), raffinose, trehalose and dextrates, sugar alcohols such as mannitol, sorbitol, maltitol, xylitol and lactitol, compressible sugar, microcrystalline cellulose, powdered cellulose, siliconized microcrystalline cellulose, calcium hydrogen phosphate, calcium carbonate, calcium lactate and mixtures thereof. Microcrystalline cellulose and water-soluble fillers are preferred. Water-soluble fillers are particularly preferred.

The composition preferably comprises 5 to 60 wt. %, more preferably 10 to 50 wt. %, most preferably 20 to 50 wt. % of filler.
The term "water-soluble filler" as used herein refers to a filler having a solubility in water at 25°C of at least 0.03 g/L. Examples of water-soluble fillers include monosaccharides and oligosaccharides such as glucose, fructose, saccharose, lactose (anhydrous and monohydrate,), raffinose, trehalose and dextrates, sugar alcohols such as mannitol, sorbitol, maltitol, xylitol and Lactitol. Lactose, mannitol, sorbitol or xylitol are particularly preferred. It is particularly preferred that the composition comprises at least 20 wt. %, particularly 20 to 50 wt. %, more preferably 20 to 45 wt. %, most preferably 25 to 45 wt. % of at least one water-soluble filler.

The composition can also include at least one water-insoluble filler. The term "water-insoluble filler" as used herein refers to a filler having a solubility in water at 25°C of less than 0.03 g/L. Examples of water-insoluble fillers include microcrystalline cellulose, powdered cellulose, siliconized microcrystalline cellulose, calcium hydrogen phosphate, calcium carbonate, calcium lactate and mixtures thereof. Microcrystalline cellulose and calcium hydrogen phosphate are particularly preferred. It is preferred that the composition comprises 5 to 50 wt. %, more preferably 10 to 40 wt. %, most preferably 15 to 30 wt. % of at least one water-insoluble filler.

It is further preferred that the composition comprises a water-soluble filler and a water-insoluble filler in a weight ratio of 1:1 to 10:1, particularly 1:1 to 5:1, most preferably 1.2:1 to 9.5:1.

Suitable binders include povidone (polyvinylpyrrolidone), copovidone (vinylpyrrolidone-vinylacetate copolymer), microcrystalline cellulose, powdered cellulose, crystalline cellulose, siliconized microcrystalline cellulose, cellulose derivatives such as hydroxymethylcellulose, hydroxyethylcellulose, hydroxypropylcellulose and hydroxypropylmethylcellulose, starch, pregelatinized starch, polymethacrylates, compressible sugars, sucrose and sugar alcohols such as mannitol, sorbitol, maltitol and xylitol and mixtures thereof. Preferred are povidone, copovidone, mannitol and sorbitol.

According to a preferred embodiment, the binder comprises at least one low-melting binder having a melting point below 120°C, particularly below 100°C and most preferably below 80°C. Suitable low-melting binders include polyethyleneglycols having an average molecular weight of below 15,000 and particularly in the range of 1,500 to 10,000, hydrogenated castor oil, stearic acid and stearic alcohol. Binders having a melting point below 80°C and/or having surfactant properties as a ionic surfactant are particularly preferred. As used herein, the term "having surfactant properties" refers to a substance decreasing the surface tension of water by at least 20 % in comparison to pure water at 25°C when used at a concentration of 0.1 g/L or less. Low-melting binders and binders having surfactant properties as ionic surfactants are particularly suitable for use in hot melt processes.

The composition preferably comprises 0.5 to 40 wt. %, particularly 1 to 20 wt. %, more preferably 2 to 10 wt. %, most preferably 3 to 7 wt. % of binder.

The term "disintegrant" as used herein refers to any material that has wicking and/or swelling properties when it comes in contact with water. Suitable disintegrants include povidone, crospovidone, starch, pregelatinized starch, sodium starch glycolate, hydroxypropyl starch, microcrystalline cellulose, carboxymethylcellulose sodium or calcium, croscarmellose, polacrilin potassium, low-substituted hydroxypropylcellulose, sodium or calcium alginate, docusate sodium, methylcellulose, agar, guar gum, chitosan, alginic acid and mixtures thereof. Preferred are crospovidone and croscarmellose. The composition preferably comprises 1 to 20 wt. %, particularly 2 to 10 wt. %, more preferably 3 to 8 wt. %, most preferably 4 to 7 wt. % of disintegrant.

Suitable lubricants include fatty acids such as stearic acid, palmitic acid and oleic acid, fatty acid salts such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. The composition preferably comprises 0.1 to 10 wt. %, particularly 0.25 to 5 wt. %, more preferably 0.5 to 2 wt. % of lubricant.

Suitable glidants include colloidal silicon dioxide and magnesium trisilicate. The composition preferably comprises 0.1 to 10 wt. %, particularly 0.25 to 5 wt. %, more preferably 0.5 to 2 wt. % of glidant.

Suitable coloring agents include dyes and pigments such as iron oxide and titanium oxide. The composition preferably comprises 0.001 to 1 wt. %, particularly 0.01 to 0.5 wt. %, more preferably 0.05 to 0.2 wt. % of the coloring agent.

The composition is optionally film coated. Film coating can be used to provide for improved surface smoothness and color, increased chemical and physical stability of the active agent due to reduced permeability for gases such as oxygen and/or water vapor, less disintegration of the solid composition in acidic medium resulting in decreased gastrointestinal side effects, and easier swallowing of the tablet. The film coating comprises coating materials generally known in the art. Suitable coating materials include polymers such as hydroxypropylcellulose, hydroxypropylmethylcellulose, ethylcellulose, povidone and copovidone, graft copolymers of polyethyleneglycol and polyvinyl alcohol (Kollicoat IR), shellac, polymers of methacrylic acid or methacrylic acid esters, methacrylic acid-methyl acrylate copolymers, polyvinyl alcohol, plasticizers such as propylene glycol, polyethylene glycol, glycerol triacetate, triethyl citrate, antitacking agents such as talc, glycerol monostearate, magnesium stearate and colorants or pigments such as titanium dioxide or iron oxide.

In a third embodiment a process for the hydrophilization of the hydrophobic active ingredient may be applied. Surprisingly, it has now been found that a special treatment of the surface of the active compound in the course of the moist granulation brings about improved solubility and subsequently absorption behavior. The use of the active compound in hydrophilized form in the preparation of solid, orally administrable pharmaceutical compositions leads to a significant increase in the dissolution profile of the formulation thus obtained.

The process of this embodiment of the invention comprises the following steps:
(a) first granules comprising the active compound dronedarone or its pharmaceutically acceptable salt, preferably dronedarone hydrochloride, are prepared by moist granulation,
(b) and the granules are then converted into the pharmaceutical composition, if appropriate with addition of pharmaceutical suitable additives.

The moist granulation in process step (a) can be carried out under high shear (=mixer granulation) or in a fluidized bed (=fluidized bed granulation); fluidized bed granulation is preferred. Under moist granulation the granulation with water, an organic solvent or a granulation liquid comprised of a molten binder (hot melt granulation) may be understood. If the volume of the granulation liquid is small compared to the amount of excipients and/or active ingredient in dry from, preferably less than 0.04 mL per g of the amount of excipients in dry form, preferably the term moisture activated dry granulation is applied (MADG).

In the moist granulation, the active compound can either be introduced into the pre-mixture (original mixture) as a solid or it is suspended in the granulating liquid. Preferably, the active compound suspended in the granulating liquid is introduced into the moist granulation (suspension process).

The pre-mixture (original mixture) can comprise fillers, binders or disintegrants as defined above or any mixtures thereof.

The granulating liquid used according to the invention contains a solvent, a hydrophilic binding agent, an ionic surfactant and, if appropriate, a wetting agent. The hydrophilic binding agent is in this case dispersed in the granulating liquid or preferably dissolved therein. The solvents used for the granulating liquid can be organic solvents, such as, for example, ethanol or acetone, or water or mixtures thereof. Preferably, water is used as a solvent. The hydrophilic binding agents employed for the granulating liquid are pharmaceutically suitable hydrophilic additives, preferably those which dissolve in the solvent of the granulating liquid. Preferably, hydrophilic polymers such as, for example, hydroxypropylmethylcellulose (HPMC), carboxymethylcellulose (sodium and calcium salts), ethylcellulose, methylcellulose, hydroxyethylcellulose, ethylhydroxyethylcellulose, hydroxypropylcellulose (HPC), L-HPC (low-substituted HPC), polyvinylpyrrolidone, polyvinyl alcohol, polymers of acrylic acid and its salts, vinylpyrrolidone-vinyl acetate copolymers (for example Kollidon(R) VA64, BASF), gelatine, guar gum, partially hydrolysed starch, alginates or xanthan are employed here.

The hydrophilic binding agent can be present here in a concentration of 1 to 15% (based on the total mass of the pharmaceutical composition), preferably of 1 to 8%.

The process can further comprise micronization of dronedarone hydrochloride to obtain dronedarone hydrochloride in micronized form with particle size d₉₀<20 µm, preferably d₉₀<15 µm, more preferably d₉₀ < 6 micrometers and/ or its specific surface area more than 1 m²/ g, preferably more than 2,5 m²/g, most preferably more than 5 m²/g. Of course, if the above second variant of the first embodiment is carried out, micronization may be effected to yield the particles sizes and/or specific surface areas as characterized above for this variant. The process according to this embodiment may also be combined with any other embodiment or variant of an embodiment as described above. Micronization of dronedarone hydrochloride is performed in the dry state using dry mills such as cutting mills, pin/cage mills, hammer mills, jet mills, fluidized bed jet mills and ball mills. It is particularly preferred that micronized dronedarone hydrochloride is used in step (a).

Step (a) can also comprise hot melt granulation of dronedarone hydrochloride with at least one excipient and optionally mixing the obtained granulate with additional excipients. Hot melt granulation can be performed by granulating dronedarone hydrochloride with at least one excipient in a granulating machine such as a high-shear mixer at a temperature of at least the melting point of at least one excipient. Preferably, dronedarone hydrochloride is granulated with at least one low-melting binder having a melting point below 120°C, particularly below 100°C, most preferably below 80°C, at a temperature of at least the melting point of said binder. Preferred low-melting binders are those defined above for the first aspect of the invention.

Hot melt granulation can also be performed by heating at least one excipient to at least its melting point, dispersing dronedarone hydrochloride and optionally further excipients in the molten excipient and spraying the dispersion onto at least one further excipient. Preferably, the molten excipient is a low-melting binder as described above. In particular, dronedarone hydrochloride and an ionic surfactant can be dispersed in molten binder and the dispersion sprayed onto a mixture of filler and optionally disintegrant. The obtained mixture is typically cooled, milled into granules and sieved.

According to another embodiment, step (a) comprises hot melt extrusion of the active pharmaceutical ingredient with at least one excipient, milling the extrudate and optionally mixing the milled extrudate with additional excipients. Extrusion can be performed using conventional extruders. The obtained extrudate is milled for instance using a jet mill. Preferably, dronedarone or its pharmaceutically acceptable salt is extruded with at least one low-melting binder having a melting point below 120°C, particularly below 100°C, most preferably below 80°C, at a temperature of at least the melting point of said binder. Preferred low-melting binders are those defined above for the first aspect of the invention.

In step (b), the mixture is preferably compressed to give a tablet. The obtained tablet is optionally film-coated according to coating methods generally known in the art.

The last embodiment according to the present invention relates to the preparation of granulation liquid which comprises de-agglomeration and/or milling and/or micronization of active ingredient. Optionally it can comprise de-agglomeration and/or co-milling and/or co-micronization of active ingredient with at least one further excipient. The granulation liquid obtained according to this embodiment may be used for manufacturing pharmaceutical compositions. This includes especially the pharmaceutical compositions according to any one of the embodiments and variants of embodiments described above.

The process can be carried out in various wet homogenizers/micronizers. This term refers to any known laboratory and/or industry scale device. Preferred wet homogenizers/micronizers are high pressure homogenizers/micronizers, such as homogenizers produced by APV. Wet pearl grinders, such as Hosokawa Alpine agitated ball mill, ultrasound processors, such as Hielscher ultrasonic processor, and in particular high shear mixers and colloid mills, such as Turrax and colloid mills produced by Fryma.

According to the last embodiment of the present invention a process of preparing a granulation liquid comprises the following steps:
i) suspending the active ingredient in a pharmaceutically acceptable solvent
ii) optionally addition of further excipients
iii) homogenizing and/or micronization of the suspension

The solvent can be selected from water, alcohols (such as for example methyl alcohol, ethyl alcohol, propyil alcohol, i-propyl alcohol etc), acetone, any other pharmaceutically acceptable solvent or mixtures thereof. Preferably, water or alcohols or mixtures thereof are used. Most preferably water is used as a solvent.

Further excipients can be selected from a group of surfactants, solubilizers, binders, antifoaming agents, stabilizers, plasticizers, pH modifiers, colouring agents etc.

As a binder any water soluble pharmaceutically acceptable polymer can be used. It can be selected but not limited to the group of cellulose esters and ethers (such as hydroxypropyl cellulose, hydroxypropyl methylcellulose with viscosity of 2 w/vol% aqueous solution according to method of Ph. Eur. of less than 20 mPas, preferably of less than 10mPas, methylcellulose, carboxymethyl cellulose etc.), povidone with K values of less than 50, preferably of less than 35, acacia, polyethylene glycol etc.

Dimethylpolysiloxane (dimeticone) or other antifoaming agent can be used to prevent foaming of the granulation liquid.

Polyethylene glycols, triethylcitrate, tributylcitrate, triacetine, dibutylsebacate etc. can be used as plasticizers.

Optionally titanium dioxide, iron oxides and other coloring agents can also be applied.

It has surprisingly been found that tablets prepared according to the above processes exhibit a good dissolution profile and bioavailability while offering important advantages in terms of process economy and suitability for industrial application.

All compositions according to the invention can be packaged into various kinds of containers. Thus, the invention also relates to a packaged pharmaceutical composition comprising the solid pharmaceutical composition described above packaged in a container made of glass or polypropylene with or without dessicant or in a blister made of single or multiple polymer and/or aluminium layers. According to a particular embodiment, the packaged composition is packaged with an atmosphere having a reduced oxygen concentration such as below 15 vol. %, particularly below 10 vol. %, more preferably below 5 vol. % oxygen. According to a particularly preferred embodiment, the composition is packaged into a nitrogen atmosphere.

The invention also relates to a solid pharmaceutical composition prepared by the process according to the invention.

### Examples

### Example 0

Dronedarone hydrochloride used in the pharmaceutical composition according to present invention can be prepared by any method known from the state of the art, such as for example: Saravanan Mohanarangam et al, J. Chin. Chem. Soc., Vol. 58, No 6, 2011; WO 2011/153923; WO 03/048144; US 2005/049302; EP 0 471 609 A2; EP 2 371 808 A1; US 7,312,346; EP 1 343 777; WO 2009/044143; WO 2010/040261; CN 101153012 B; CN 101838252 A1; EP 1 394 155 A1; WO 2008/15221 A2

The last step, crystallization step can be performed according to the following examples:

### Example 0.1: Crystallization from acetone:

Dronedarone hydrochloride (500g, HPLC: 98,7 area%) was dissolved in acetone (8L) at reflux temperature. To the clear solution active carbon is added and solution is filtered in 10L reactor. Solution is kept hot and 950 mL of acetone is removed by destilation. Clear solution is cooled to 45 °C and seeded with 5 g of dornedaron hydrochloride. Obtained suspension was cooled to 0 °C in 5 hours, stirred at 0 °C until crystallization was complete. Material was collected and dried in vacuum dryer at 35 °C. Yieid: 450 g, HPLC: 99,5 area%. Dry material was micronized with Jetmill MC-50 at high feeding rate.

### Example 0.2: Crystallization from isopropanole:

In 10 L reactor dronedarone hydrochloride (1 kg) was dissolved in 6 L of isopropanole at reflux temperature. Clear solution was cooled to 60 °C and seeded with 5 g of dronedarone hydrochloride and stirred for 30 minutes. Obtained suspension was cooled to 0 °C in 5 hours, stirred at 0 °C until crystallization was complete. Material was collected and dried in vacuum dryer at 35 °C. Yield: 450 g. Dry material was micronized with Jetmill MC-50 at high feeding rate

### Example 0.3: Crystallization from acetic acid/isopropanole mixture:

Dronedarone hydrochloride (200 g) was dissolved in 200 mL acetic acid at 40 °C. 10g of active carbon is added and stirred for 30 minutes and solution was filtered to 5 L reactor. To the filtered solution 2L of izopropilacetate was added at room temperature. Obtained suspension was stirred 1 hour at 25 °C and cooled to 0°C in 1 hour. Suspension was stirred at 0 °C until crystallization was complete. Material was collected and dried in vacuum dryer at 35 °C. Yield: 450 g. Dry material was micronized with Jetmill MC-50 at high feeding rate.

### Example 0.4: Crystalilization from isopropanole (overmicronized):

Dronedarone hydrochloride (200 g) was dissolved in 1200 mL of isopropanole at reflux temperature. 10 g of active carbon was added to the solution, stirred at reflux and filtered into 2 L reactor. Clear solution was cooled to 60 °C and seeded with 5g of dronaderone hydrochloride and stirred for 30 minutes. Obtained suspension was cooled to 0 °C in 5 hours, stirred at 0 °C until crystallization was complete. Material was collected and dried in vacuum dryer at 35 °C. Yield: 186.8 g. Dry material was micronized with Jetmill MC-50 at very low feeding rate.

The resulting dronedarone particles can be characterized as follows:

| | Specific surface area (m²/g) | Particle size d₉₀ (µm) |
|---|---|---|
| Overmicronized dronedarone HCl - API 1- Example 0.4 | 8.3 | 5.1 |
| Micronized dronedarone HCl - Example 0.1 - API 2 | 6.2 | 7.1 |
| Micronized dronedarone HCl - -Example 0.2 - API 3 | 6.0 | 7.4 |

### Example 1

### Micronization of dronedarone hydrochloride:

Dronedarone hydrochloride was micronized using an air-jet mill with an inlet air pressure of about 9 bar and a milling pressure of about 8.5 bar.

### Example 2

### Co-milling of dronedarone hydrochloride together with hydrophilic excipient

50 g of dronedarone hydrochloride and 10 g of lactose were co-milled using an air-jet mill with an inlet air pressure of about 9 bar and a milling pressure of about 8.5 bar.

### Example 3

### Preparation of tablets by direct compression

Tablet compositions were prepared as follows:

| | **3A** | **3B** | **3C** | **3D** | **3E** | **3F** | **3G** | **3H** |
|---|---|---|---|---|---|---|---|---|
| Dronedarone hydrochloride | 426.0 | 426.0 | 426.0 | 426.0 | 426.0 | 426.0 | 426.0 | 426.0 |
| Sodium lauryl sulfate | 12.0 | 12.0 | 12.0 | 12.0 | | | | |
| Sodium docusate | | | | | 17.0 | 17.0 | 17.0 | 17.0 |
| Mannitol | 102.5 | 102.5 | | | 97.5 | 97.5 | | |
| Lactose spraydried | | | 102.5 | 98.5 | | | 97.5 | 93.5 |
| Cellulose, microcrystalline | 30.0 | | | 30.0 | | | | 30.0 |
| Calcium hydrogen phosphate | | 30.0 | 30.0 | | | 30.0 | 30.0 | |
| Crospovidone | 28.0 | 28.0 | 28.0 | | 28.0 | 28.0 | 28.0 | |
| Croscarmellose | | | | 28.0 | | | | 28.0 |
| Magnesium stearate | 1.5 | 1.5 | 1.5 | 1.5 | 1.5 | 1.5 | 1.5 | 1.5 |
| Total weight (mg) | 600.0 | 600.0 | 600.0 | 600.0 | 600.0 | 600.0 | 600.0 | 600.0 |

Micronized dronedarone hydrochloride according to example 1 was dry-mixed with the other excipients. The resulting mixture was directly compressed to form tablets.

### Example 4

### Reparation of tablets _by dry granulation

Tablet compositions were prepared as follows:

| | **4A** | **4B** | **4C** | **4D** | **4E** | **4F** | **4G** | **4H** |
|---|---|---|---|---|---|---|---|---|
| Dronedarone hydrochloride | 426.0 | 426.0 | 426.0 | 426.0 | 426.0 | 426.0 | 426.0 | 426.0 |
| Sodium lauryl sulfate | 12.0 | 12.0 | 12.0 | 12.0 | | | | |
| Sodium docusate | | | | | 17.0 | 17.0 | 17.0 | 17.0 |
| Mannitol | 101.5 | 101.5 | | | 96.5 | 96.5 | | |
| Lactose spray-dried | | | 101.5 | 97.5 | | | 96.5 | 92.5 |
| Cellulose, microcrystalline | 30.0 | | | 30.0 | 30.0 | | | 30,0 |
| Calcium hydrogen phosphate | | 30.0 | 30.0 | | | 30.0 | 30.0 | |
| Crospovidone | 28.0 | 28.0 | 28.0 | | 28.0 | 28.0 | 28.0 | |
| Croscarmellose | | | | 28.0 | | | | 28.0 |
| Magnesium stearate | 1.0 | 1.0 | 1.0 | 1.0 | 1.0 | 1.0 | 1.0 | 1.0 |
| Magnesium stearate | 1.5 | 1.5 | 1.5 | 1.5 | 1.5 | 1.5 | 1.5 | 1.5 |
| Total mass (mg) | 600.0 | 600.0 | 600.0 | 600.0 | 600.0 | 600.0 | 600.0 | 600.0 |

Dronedarone hydrochloride was mixed with the other excipients, sieved and mixed again. The mixture was dry granulated with a roller-compactor using a Fitzpatrick laboratory machine. The resulting compact was milled into granules, mixed with the second part of the Magnesium stearate and compressed into tablets.

### Example 5

### Preparation of tablets by hot melt granulation

Tablet compositions were prepared as follows:

| | **5A** | **5B** | **5C** | **5D** |
|---|---|---|---|---|
| Dronedarone hydrochloride | 426.0 | 426.0 | 426.0 | 426.0 |
| Stearic alcohol | 80.0 | 80.0 | 80.0 | |
| Polyethylene glycol (e.g. PEG 6000) | | | | 80.0 |
| Sodium lauryl sulfate | 12.0 | 12.0 | 12.0 | 12.0 |
| Mannitol | 99.0 | 101.5 | | 101.5 |
| Maize starch | | | 101.5 | |
| Crospovidone | 8.0 | 6.0 | 6.0 | 6.0 |
| Cellulose, microcrystalline | 14.0 | 14.0 | 14.0 | 14.0 |
| Colloidal silicon dioxide | 0.5 | 0.5 | 0.5 | 0.5 |
| Sodium stearil fumarate | 0.5 | 0.5 | 0.5 | 0.5 |
| Total mass (mg) | 640.0 | 640.0 | 640.0 | 640.0 |

Dronedarone hydrochloride was mixed with the other excipients (except colloidal silicon dioxide and sodium stearil fumarate) in a high-shear granulation machine at a temperature of about 50-70°C for about 5 to 15 minutes. The mixture was cooled down to ambient temperature and mixed with sodium stearil fumarate and colloidal silicon dioxide. The resulting mixture was compressed into tablets.

### Example 6

### Preparation of tablets by granulation with melted binder

Tablet compositions were prepared as follows:

| | **6A** | **6B** |
|---|---|---|
| | | |
| Dronedarone hydrochloride | 426.0 | 426.0 |
| Stearic alcohol | 80.0 | 40.0 |
| Mannitol (Pearlitol 200 SD) | 40.0 | 80.0 |
| Sodium lauryl sulfate | 12.0 | 12.0 |
| Crospovidone | 5.0 | 5.0 |
| Avicel PH 200 | 15.0 | 15.0 |
| Mannitol (Pearlitol 200 SD) | 53.5 | 55.5 |
| Crospovidone | 6.0 | 4.0 |
| Aerosil 200 | 1.0 | 1.0 |
| Magnesium stearate | 1.5 | 1.5 |
| | | |
| Total mass (mg) | 642.0 | 642.0 |

Dronedarone hydrochloride and sodium lauryl sulfate were dispersed in the melted binder (stearic alcohol). The dispersion was sprayed onto a mixture of a first amount of mannitol (Pearlitol 200 SD) and crospovidone using adapted laboratory fluid bed equipment Glatt GPCG3. The obtained dispersion was cooled, milled and sieved through 1 mm sieve. The sieved dispersion was mixed with Avicel and the remaining amounts of mannitol and crospovidone and finally with Aerosil and magnesium stearate and then compressed into tablets.

### Example 7

### Preparation of tablets fluidized bed granulation process

Tablet compositions was prepared as follows:

| | **7A** |
|---|---|
| Dronedarone hydrochloride, micronized | 426.0 |
| Maize starch | 47.2 |
| Lactose monohydrate | 60.0 |
| Crospovidone | 69.0 |
| Hypromellose | 20.0 |
| Sodium lauryl sulphate (Texapon K12) | 12.8 |
| Colloidal anhydrous silica | 2.0 |
| Magnesium stearate | 3.0 |
| Total mass (mg) | 640.0 |

Hypromellose and sodium lauryl sulphate were dissolved in water. The micronized active compound was suspended in this solution. The suspension thus prepared was sprayed onto the original mixture of maize starch, lactose monohydrate and crospovidone as granulation liquid in the course of fluidized bed granulation. After drying and sieving the resulting granules, the colloidal anhydrous silica and magnesium stearate were added and mixed.

### Example 8

### Reparation of tablets using fluidized bed granulation process

Tablet compositions was prepared as follows:

| | **8A** |
|---|---|
| Dronedarone hydrochloride, micronized | 426.0 |
| Maize starch | 47.2 |
| Lactose monohydrate | 69.0 |
| Crospovidone | 69.0 |
| Hypromellose | 20.0 |
| Benzalkonium chloride | 3.8 |
| Colloidal anhydrous silica | 2.0 |
| Magnesium stearate | 3.0 |
| Total mass (mq) | 640.0 |

Hypromellose and benzalkonium chloride were dissolved in water. The micronized active compound was suspended in this solution. The suspension thus prepared was sprayed onto the original mixture of maize starch, lactose monohydrate and crospovidone as granulation liquid in the course of fluidized bed granulation. After drying and sieving the resulting granules, the colloidal anhydrous silica and magnesium stearate were added and mixed.

### Example 9

### Preparation of tablets using high shear granulation process

Tablet compositions was prepared as follows:

| | **9A** | **9B** | **9C** |
|---|---|---|---|
| Dronedarone hydrochloride, micronized | 426.0 | 426.0 | 426.0 |
| Sodium lauryl sulphate | 14.0 | | |
| Sodium docusate | | 20.3 | |
| Benzalkonium chloride | | | 4.7 |
| Maize starch | 33.0 | 33.0 | 33.0 |
| Lactose monohydrate | 155.0 | 148.7 | 164.3 |
| Crospovidone | 30.0 | 30.0 | 30.0 |
| Maize starch | 32.0 | 32.0 | 32.0 |
| Colloidal anhydrous silica | 4.0 | 4.0 | 4.0 |
| Magnesium stearate | 6.0 | 6.0 | 6.0 |
| Total mass (mg) | 700.0 | 700.0 | 700.0 |

Dronedarone hydrochloride, micronized, sodium lauryl sulphate/sodium docusate/benzalkonim chloride, lactose monohydrate, maize starch and crospovidone were mixed in a high speed mixer. The purified water was added to the original granule mixture as a granulation liquid and blended uniformly with the original granule mixture with the aid of the rapidly rotating stirrer. After mixing has been carried out, the moist granules were sieved and dried in the fluidized bed. After sivieng the dried granules, the maize starch and colloidal silica were added and mixed. Additionally the magnesium starate were added and mixed.

### Example 10

### Preparation of tablets using high shear granulation rocess

Tablet compositions was prepared as follows:

| | **10A** | **10B** | **10C** |
|---|---|---|---|
| Dronedarone hydrochloride, micronized | 426.0 | 426.0 | 426.0 |
| Sodium lauryl sulphate | 14.0 | | |
| Sodium docusate | | 20.3 | |
| Benzalkonim chloride | | | 4.7 |
| Mannitol | 62.0 | 62.0 | 62.0 |
| Lactose monohydrate | 127.0 | 120.7 | 136.3 |
| Crospovidone | 30.0 | 30.0 | 30.0 |
| Maize starch | 32.0 | 32.0 | 32.0 |
| Colloidal anhydrous silica | 4.0 | 4.0 | 4.0 |
| Magnesium stearate | 5.0 | 5.0 | 5.0 |
| Total mass (mg) | 700.0 | 700.0 | 700.0 |

Sodium lauryl sulphate/sodium docusate/benzalkonim chloride was dissolved in purified water and added to mannitol as a granulation liquid in the high shear mixer. After mixing has been carried out, the moist granules were sieved and dried in the fluidized bed. After sieving the dried granules, the dronedarone hydrochloride, micronized, maize starch, lactose monohydrate and crospovidone were added and mixed. Additionally colloidal anhydrous silica and magnesium stearate were added and mixed.

### Example 11

### Reparation of tablets using high shear granulation process

Tablet compositions were prepared as follows:

| | **11A** | **11B** | **11C** |
|---|---|---|---|
| Dronedarone hydrochloride, micronized | 426.0 | 426.0 | 426.0 |
| Lactose monohydrate | 85.2 | 85.2 | 85.2 |
| Sodium lauryl sulphate | 14.0 | | |
| Sodium docusate | | 20.3 | |
| Lecithin | | | 43.0 |
| Mannitol | 62.0 | 62.0 | 62.0 |
| Lactose monohydrate | 41.8 | 35.5 | 35.5 |
| Crospovidone | 30.0 | 30.0 | 30.0 |
| Maize starch | 32.0 | 32.0 | 32.0 |
| Colloidal anhydrous silica | 4.0 | 4.0 | 4.0 |
| Magnesium stearate | 5.0 | 5.0 | 5.0 |
| Total mass (mg) | 700.0 | 700.0 | 700.0 |

Sodium lauryl sulphate/Sodium docusate/Lecithin was dissolved in a purified water (in the case of the lecithin the ethanol was used) and added to the mannitol as a granulation liquid in the high shear mixer. After mixing has been carried out, the moist granules were sieved and dried in the fluidized bed. After sieving the dried granules, the co-milled dronedarone hydrochloride prepared according to example 2, maize starch, lactose monohydrate and crospovidone were added and mixed. Additionally colloidal anhydrous silica and magnesium stearate were added and mixed.

### Example 12

### Preparation of tablets usin fluid bed granulation process

Tablet compositions was prepared as follows:

| | **12A** | **12B** |
|---|---|---|
| Dronedarone hydrochloride, micronized | 426.0 | 426.0 |
| Sodium lauryl sulphate | 16.0 | |
| Sodium docusate | | 23.2 |
| PVP | 65.0 | 65.0 |
| Mannitol | 196.0 | 188.8 |
| Avicel PH102 | 93.0 | 93.0 |
| Magnesium stearate | 4.0 | 4.0 |
| Total mass (mg) | 800.0 | 800.0 |

Dronedarone hydrochloride (micronized) was added to the aqueous solution of sodium lauryl sulphate/sodium docusate and PVP. The suspension thus prepared was sprayed onto the lactose monohydrate as granulation liquid in the course of fluidized bed granulation. After drying and sieving the resulting granules, Avicel PH102 and magnesium stearate were added and mixed.

### Example 13

**Preparation of tablets comprising dronaderone by spray granulation**

| | **13A** |
|---|---|
| Dronedarone hydrochloride, micronized | 426.0 |
| Sodium lauryl sulphate | 17.0 |
| PVP | 20.0 |
| Lactose monohydrate | 197.0 |
| Microcrystalline cellulose (Avicel PH101) | 92.0 |
| Croscarmellose sodium | 24.0 |
| Microcrystalline cellulose (Avicel PH102) | 100.0 |
| Magnesium stearate | 4.0 |
| Total mass (mg) | 880.0 |

Povidone and sodium lauryl sulphate were added to dronaderone hydrochloride suspensions. This suspension was then treated with a high pressure homogenizer APV 2000 (3 cycles at 750 bar). The suspension was then sprayed onto the powder mixture of lactose monohydrate, microcrystalline cellulose and croscarmellose sodium. The obtained granulate was dried and sieved, the remaining excipients were added and the mixture was compressed into tablets.

### Example 14

### Preparation of tablets using fluid bed granulation process

Tablet compositions was prepared as follows:

| | **14A** |
|---|---|
| Dronedarone hydrochloride, micronized | 426.0 |
| Lecithin | 43.0 |
| PVP | 65.0 |
| Mannitol | 169.0 |
| Avicel PH102 | 93.0 |
| Magnesium stearate | 4.0 |
| Total mass (mg) | 800.0 |

Dronedarone hydrochloride (micronized) was added to the alcohol solution of lecithin and PVP. The suspension thus prepared was sprayed onto the lactose monohydrate as granulation liquid in the course of fluidized bed granulation. After drying and sieving the resulting granules, the Avicel PH102 and magnesium stearate were added and mixed.

### Example 15

### Preparation of tablets using high shear granulation process

Tablet compositions was prepared as follows:

| | **15A** |
|---|---|
| Dronedarone hydrochloride, micronized | 426.0 |
| Lecithin | 43.0 |
| Maize starch | 30.0 |
| Lactose monohydrate | 134.0 |
| Crospovidone | 30.0 |
| Maize starch | 27.0 |
| Colloidal anhydrous silica | 4.0 |
| Magnesium stearate | 6.0 |
| Total mass (mg) | 700.0 |

Dronedarone hydrochloride (micronized), lecithin, lactose monohydrate, maize starch and crospovidone were mixed in a high speed mixer. Purified water was added to the original granule mixture as a granulation liquid and blended uniformly with the original granule mixture with the aid of a rapidly rotating stirrer. After mixing has been carried out, the moist granules were sieved and dried in the fluidized bed. After sieving the dried granules, the maize starch and colloidal silica were added and mixed. Additionally magnesium starate was added and mixed.

### Example 16

### Preparation of tablets using granules comprising the active compound and different surfactants

Tablet compositions were prepared as follows:

| | **16A** | **16B** | **16C** | **16D** | **16E** |
|---|---|---|---|---|---|
| Dronedarone hydrochloride, micronized | 426.0 | 426.0 | 426.0 | 426.0 | 426.0 |
| Maize starch | 45.5 | 45.5 | 45.5 | 45.5 | 45.5 |
| Lactose monohydrate | 41.65 | 77.23 | 41.65 | 41.65 | 37.23 |
| Crospovidone | 65.0 | 65.0 | 65.0 | 65.0 | 65.0 |
| Hypromellose | 26.0 | 26.0 | 26.0 | 26.0 | 26.0 |
| Colloidal anhydrous silica | 2.6 | 2.6 | 2.6 | 2.6 | 2.6 |
| Gelucire 50/13 | 40.0 | | | | |
| Benzalkonium chloride | | 4.42 | | | 4.42 |
| Polysorbate 80 | | | 40.0 | | |
| Phospholipone 90 H | | | | 40.0 | |
| Phospholipone 90 G | | | | | 40.0 |
| Magnesium stearate | 3.25 | 3.25 | 3.25 | 3.25 | 3.25 |
| Total mass (mg) | 650.0 | 650.0 | 650.0 | 650.0 | 650.0 |

Dronedarone hydrochloride, methylhydroxypropylcellulose, lactose monohydrate, maize starch and crospovidone were mixed in a high speed mixer.
The obtained mixture was granulated with a solution of surfactant: Gelucire, benzalkonium chloride or Tween 80. The moist granules were sieved and dried in the fluidized bed. After sieving the dried granules, the colloidal silica and magnesium stearate were added and mixed. The final mixture is then tabletted in a proportion of 650 mg per unit.

Dronedarone hydrochloride, methylhydroxypropylcellulose, lactose monohydrate, maize starch, crospovidone and a half amount of Phospholipon 90 H were mixed in a high speed mixer.
The obtained mixture was granulated with a solution of a half amount of Phospholipon 90 H. The moist granules were sieved and dried in the fluidized bed. After sieving the dried granules, the colloidal silica and magnesium stearate were added and mixed. The final mixture is then tabletted in a proportion of 650 mg per unit.

Dronedarone hydrochloride, methylhydroxypropylcellulose, lactose monohydrate, maize starch and crospovidone were mixed in a high speed mixer.
The obtained mixture was granulated with an alcohol solution of Phospholipon 90 G and benzalkonium chloride. The moist granules were sieved and dried in the fluidized bed. After sieving the dried granules, the colloidal silica and magnesium stearate were added and mixed. The final mixture is then tabletted in a proportion of 650 mg per unit.

### Example 17

### Preparation of tablets using granules comprising the active compound and a surfactant

Tablet compositions was prepared as follows:

| | **17A** |
|---|---|
| Dronedarone hydrochloride, micronized | 426.0 |
| Maize starch | 45.5 |
| Lactose monohydrate | 41.65 |
| Crospovidone | 65.0 |
| Hypromellose | 26.0 |
| Colloidal anhydrous silica | 2.6 |
| Poloxamer 407 | 40.0 |
| Magnesium stearate | 3.25 |
| Total mass (mg) | 650.0 |

Dronedarone hydrochloride, lactose monohydrate, maize starch, crospovidone and Poloxamer 407 were mixed in a high speed mixer.
The obtained mixture was granulated with a solution of methylhydroxypropylcellulose. The moist granules were sieved and dried in the fluidized bed. After sieving the dried granules, the colloidal silica and magnesium stearate were added and mixed. The final mixture is then tabletted in a proportion of 650 mg per unit.

### Example 18

### Preparation of tablets using granules without surfactant

Tablet compositions was prepared as follows:

| | **18A** |
|---|---|
| Dronedarone hydrochloride, micronized | 426.0 |
| Maize starch | 45.5 |
| Lactose monohydrate | 81.65 |
| Crospovidone | 65.0 |
| Hypromellose | 26.0 |
| Colloidal anhydrous silica | 2.6 |
| Magnesium stearate | 3.25 |
| Total mass (mg) | 650.0 |

Dronedarone hydrochloride, methylhydroxypropylcellulose, lactose monohydrate, maize starch and crospovidone were mixed in a high speed mixer.
The obtained mixture was granulated with purified water. The moist granules were sieved and dried in the fluidized bed. After sieving the dried granules, the colloidal silica and magnesium stearate were added and mixed. The final mixture is then tabletted in a proportion of 650 mg per unit.

### Example 19

### 19.1 Determination of concentration of dissolved dronedarone HCl in phosphate buffer pH 4.5

Solutions of different surfactants with concentrations 3.2mg / 10mL were prepared in phosphate buffer pH 4.5. 34.08 mg of dronedarone HCl was added to 10 mL of solution of each surfactant prepared as described above. After 2 hours of stirring on a magnetic stirrer the dispersions were filtered through 0.45 µm filter and appropriately diluted. The concentration of dissolved dronedarone HCl was determined by UV spectrophotometry. Experiments were performed at room temperature.

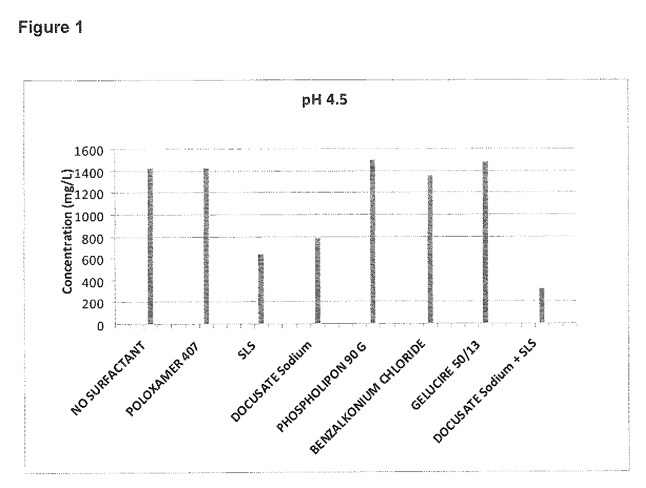

The results of this series of experiments are reported in Figure 1.

### 19.2 Determination of concentration of dissolved dronedarone HCl in phosphate buffer pH 6.7

0.5 mL of solution of dronedarone HCl in ethanol (53.3 mg of active substance / mL), 5 mL of solution of surfactant in phosphate buffer pH 4.5 (0.5 mg/mL) and 44.5 mL of phosphate buffer pH 6.8 were added in 100mL volumetric flask. The obtained dispersion was shaken for 2 hours at room temperature. The dispersion was then filtered through 0.45 filter; certain volume of initial filtrate was discarded. The concentration of dissolved dronedarone HCl was determined by UV spectrophotometry.

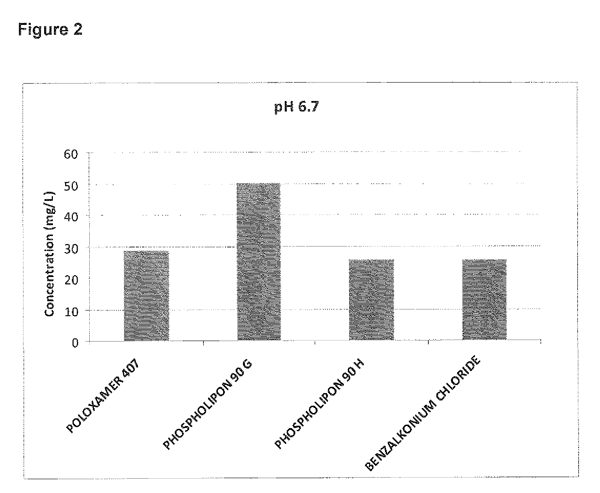

The results of this series of experiments are reported in Figure 2.

### Example 20

The dissolution profiles of several of the pharmaceutical compositions exemplified above are determined. The results of these experiments are shown in Figures 1 and 2 below.

Dissolution conditions for Dronedarone HCl 400 mg tablets:
Apparatus: **2 - Paddle** (USP and Ph. Eur.)
Rotation speed: **75 rpm**
Medium: **1000 ml Buffer Solution pH 4.5**

API 1 or API 2 was included in the pharmaceutical composition as disclosed in example 17. From Figure 1 it is clearly evident that at 60 minutes the pharmaceutical composition prepared from API 1 shows substantial lower dissolution rate in comparison to same pharmaceutical composition prepared from API 2.

The pharmaceutical compositions used in dissolution test shown in Figure 2 comprised different surfactants or no surfactant. The pharmaceutical compositions were prepared according to examples 16A-D, 17A and 18A by using API 1, API 2 or API 3.

From Figure 2 it is clearly evident that at 60 minutes the pharmaceutical composition prepared from API 1 shows substantial lower dissolution rate in comparison to other pharmaceutical compositions prepared from API 2 or API 3.

## Claims

1. A tablet comprising a pharmacologically effective amount of dronedarone or its pharmaceutically acceptable salt in an amount from 40% to 90% in weight of the active moiety based on the total weight of the tablet, **characterized in that** dronedarone or its pharmaceutically acceptable salt is micronized, optionally co-micronized together with filler, such that its specific surface area is more than 1 m²/ g and below 8 m²/g, wherein the specific surface area is determined using the BET method.

2. A tablet according to claim 1, **characterized in that** the particle size of dronedarone or its pharmaceutically acceptable salt is d₉₀<20 µm, preferably d₉₀<15 µm, more preferably d₉₀ < 6 micrometers and/ or its specific surface area is more than 2.5 m²/g, and more preferably more than 5 m²/g.

3. A tablet according to claim 1, **characterized in that** the particle size of dronedarone or its pharmaceutically acceptable salt is d₉₀ between 6 µm and 70 µm, preferably between 6 and 30 µm and more preferably between 6 and 10 µm of the active ingredient, preferably of dronedarone hydrochloride.

4. A tablet according to claims 1, 2 or 3, **characterized in that** the tablet comprises an ionic surfactant.

5. A tablet according to claims 1, 2, 3 or 4 **characterized in that** the tablet comprises dronedarone hydrochloride.

6. A tablet according to any of claims claim 1 to 5, **characterized in that** it comprises 50% to 90%, preferably 60% to 90% in weight of the active moiety based on the total weight of the tablet

7. A tablet according to any of claims 1 to 6, **characterized in that** it comprises from 1% to 20%, preferably 1% to 15% and more preferably 1% to 5% by weight of surfactant calculated on the weight of active ingredient in base form.

8. A tablet according to any of claims 1 to 6, **characterized in that** it comprises essentially no surfactant.

9. A tablet according to any of claims 1 to 7, **characterized in that** it comprises an ionic surfactant or a combination of ionic surfactants and preferably sodium lauryl sulfate, lecithin, benzalkonium chloride or sodium docusate as surfactant.

10. A tablet according to any of claims 1 to 9, **characterized in that** it contains a surfactant selected from polyethylene glycol glycerides of mono-, di- and triglycerides and mono-, and diesters of polyethylene glycol, optionally in combination with an ionic surfactant.

11. A tablet according to any of claims 1 to 10, **characterized in that** it is obtainable by moist granulation or thermoplastic granulation.

12. A tablet according to any of claims 1 to 11, **characterized in that** it is obtainable by aqueous granulation.

13. A tablet according to any of claims 1 to 12, **characterized in that** it is prepared by moisture activated dry granulation (MADG).

14. A process for the manufacture of a solid pharmaceutical composition, which is a tablet according to any one of claims 1 to 13, comprising a step of moist granulation of dronedarone or a pharmaceutically acceptable salt thereof, preferably dronedarone hydrochloride.

15. A process for the preparation of a granulation liquid which comprises de-agglomeration and/or milling and/or micronization of the active ingredient, which is selected from dronedarone and pharmaceutically acceptable salts thereof, preferably dronedarone hydrochloride, wherein said de-agglomeration and/or milling and/or micronization of the active ingredient is carried out such that its specific surface area is more than 1 m²/ g and below 8 m²/g, wherein the specific surface area is determined using the BET method, and wherein said de-agglomeration and/or milling and/or micronization of the active ingredient is optionally carried out in the presence of at least one further excipient.

## Patentansprüche

1. Tablette, umfassend eine pharmakologisch wirksame Menge von Dronedaron oder dessen pharmazeutisch annehmbares Salz in einer Menge von 40 % bis 90 % als Gewicht des wirksamen Bestandteils, bezogen auf das Gesamtgewicht der Tablette, **dadurch gekennzeichnet, dass** Dronedaron oder dessen pharmazeutisch annehmbares Salz mikronisiert, gegebenenfalls co-mikronisiert zusammen mit Füllstoff, ist, so dass seine spezifische Oberfläche mehr als 1 m²/g und unter 8 m²/g ist, wobei die spezifische Oberfläche mittels des BET-Verfahrens bestimmt wird.

2. Tablette gemäß Anspruch 1, **dadurch gekennzeichnet, dass** die Partikelgröße von Dronedaron oder dessen pharmazeutisch annehmbarem Salz d₉₀ < 20 µm ist, vorzugsweise d₉₀ < 15 µm, stärker bevorzugt d₉₀ < 6 Mikrometer ist, und/oder dessen spezifische Oberfläche größer als 2,5 m²/g, und stärker bevorzugt mehr als 5 m²/g ist.

3. Tablette gemäß Anspruch 1, **dadurch gekennzeichnet, dass** die Partikelgröße von Dronedaron oder dessen pharmazeutisch annehmbarem Salz d₉₀ zwischen 6 µm und 70 µm, vorzugsweise zwischen 6 und 30 µm und stärker bevorzugt zwischen 6 und 10 µm des Wirkstoffs ist, vorzugsweise von Dronedaronhydrochlorid ist.

4. Tablette gemäß Ansprüchen 1, 2 oder 3, **dadurch gekennzeichnet, dass** die Tablette ein ionisches Tensid umfasst.

5. Tablette gemäß Ansprüchen 1, 2, 3 oder 4, **dadurch gekennzeichnet, dass** die Tablette Dronedaronhydrochlorid umfasst.

6. Tablette gemäß einem der Ansprüche 1 bis 5, **dadurch gekennzeichnet, dass** sie 50 % bis 90 %, vorzugsweise 60 % bis 90 % nach Gewicht des aktiven Bestandteils, bezogen auf das Gesamtgewicht der Tablette, umfasst.

7. Tablette gemäß einem der Ansprüche 1 bis 6, **dadurch gekennzeichnet, dass** sie von 1 % bis 20 %, vorzugsweise 1 % bis 15 % und stärker bevorzugt 1 % bis 5 % nach Gewicht an Tensid berechnet, bezogen auf das Gewicht an Wirkstoff in Basenform, umfasst.

8. Tablette gemäß einem der Ansprüche 1 bis 6, **dadurch gekennzeichnet, dass** sie im Wesentlichen kein Tensid umfasst.

9. Tablette gemäß einem der Ansprüche 1 bis 7, **dadurch gekennzeichnet, dass** sie ein ionisches Tensid oder eine Kombination aus ionischen Tensiden und vorzugsweise Natriumlaurylsulfat, Lecithin, Bezalkoniumchlorid oder Natriumdocusat als Tensid umfasst.

10. Tablette gemäß einem der Ansprüche 1 bis 9, **dadurch gekennzeichnet, dass** sie ein Tensid, ausgewählt aus Polyethylenglykolglyceriden von Mono-, Di- und Triglyceriden und Mono- und Diestern von Polyethylenglykol, gegebenenfalls in Kombination mit einem ionischen Tensid, umfasst.

11. Tablette gemäß einem der Ansprüche 1 bis 10, **dadurch gekennzeichnet, dass** sie durch feuchte Granulierung oder thermoplastische Granulierung erhältlich ist.

12. Tablette gemäß einem der Ansprüche 1 bis 11, **dadurch gekennzeichnet, dass** sie durch wässrige Granulierung erhältlich ist.

13. Tablette gemäß einem der Ansprüche 1 bis 12, **dadurch gekennzeichnet, dass** sie durch feuchtigkeitsaktivierte Trockengranulierung (MADG) hergestellt wird.

14. Verfahren zur Herstellung einer festen pharmazeutischen Zusammensetzung, die eine Tablette gemäß einem der Ansprüche 1 bis 13 ist, umfassend einen Schritt der feuchten Granulierung von Dronedaron oder einem pharmazeutisch annehmbaren Salz davon, vorzugsweise Dronedaronhydrochlorid.

15. Verfahren zur Herstellung einer Granulierungsflüssigkeit, das Deagglomeration und/oder Mahlen und/oder Mikronisierung des Wirkstoffs, der aus Dronedaron und pharmazeutisch annehmbaren Salzen davon, vorzugsweise Dronedaronhydrochlorid, ausgewählt wird, umfasst, wobei die Deagglomeration und/oder das Mahlen und/oder die Mikronisierung des Wirkstoffs derart durchgeführt wird, dass seine spezifische Oberfläche mehr als 1 m²/g und unter 8 m²/g ist, wobei die spezifische Oberfläche mittels des BET-Verfahrens bestimmt wird, und wobei die Deagglomeration und/oder das Mahlen und/oder die Mikronisierung des Wirkstoffs gegebenenfalls in der Anwesenheit wenigstens eines weiteren Hilfsstoffes durchgeführt wird.

## Revendications

1. Comprimé comprenant une quantité efficace du point de vue pharmacologique de dronédarone, ou de son sel acceptable du point de vue pharmaceutique, en une quantité allant de 40 % à 90 % en poids de la fraction active, par rapport au poids total du comprimé, **caractérisé en ce que** la dronédarone, ou son sel acceptable du point de vue pharmaceutique, est micronisée, optionnellement co-micronisée conjointement avec une charge, de façon telle que sa surface spécifique soit supérieure à 1 m²/g et inférieure à 8 m²/g, dans lequel la surface spécifique est déterminée en utilisant le procédé BET.

2. Comprimé selon la revendication 1, **caractérisé en ce que** la taille de particule de dronédarone, ou de son sel acceptable du point de vue pharmaceutique, est d₉₀ < 20 µm, de préférence d₉₀ < 15 µm, plus préférablement d₉₀ < 6 micromètres et/ou sa surface spécifique est supérieure à 2,5 m²/g, et plus préférablement supérieure à 5 m²/g.

3. Comprimé selon la revendication 1, **caractérisé en ce que** la taille de particule de dronédarone, ou de son sel acceptable du point de vue pharmaceutique, est d₉₀ située entre 6 µm et 70 µm, de préférence entre 6 et 30 µm et plus préférablement entre 6 et 10 µm d'ingrédient actif, de préférence d'hydrochlorure de dronédarone.

4. Comprimé selon la revendication 1, 2, ou 3, **caractérisé en ce que** le comprimé comprend un tensioactif ionique.

5. Comprimé selon la revendication 1, 2, 3 ou 4, **caractérisé en ce que** le comprimé comprend de l'hydrochlorure de dronédarone.

6. Comprimé selon l'une quelconque des revendications 1 à 5, **caractérisé en ce qu'**il comprend de 50 % à 90 %, de préférence de 60 % à 90 % en poids de la fraction active, par rapport au poids total du comprimé.

7. Comprimé selon l'une quelconque des revendications 1 à 6, **caractérisé en ce qu'**il comprend de 1 % à 20 %, de préférence de 1 % à 15 % et plus préférablement de 1 % à 5 % en poids d'un tensioactif, calculé par rapport au poids de l'ingrédient actif sous forme basique.

8. Comprimé selon l'une quelconque des revendications 1 à 6, **caractérisé en ce qu'**il ne comprend sensiblement pas de tensioactif.

9. Comprimé selon l'une quelconque des revendications 1 à 7, **caractérisé en ce qu'**il comprend un tensioactif ionique ou une combinaison de tensioactifs ioniques et, de préférence du laurylsulfate de sodium, de la lécithine, du chlorure de benzalkonium ou du docusate de sodium, en tant que tensioactif.

10. Comprimé selon l'une quelconque des revendications 1 à 9, **caractérisé en ce qu'**il contient un tensioactif choisi parmi des glycérides de mono-, di- et triglycérides de polyéthylène glycol et des mono- et diesters de polyethylène glycol, optionnellement en combinaison avec un tensioactif ionique.

11. Comprimé selon l'une quelconque des revendications 1 à 10, **caractérisé en ce qu'**il est susceptible d'être obtenu par granulation humide ou par granulation thermoplastique.

12. Comprimé selon l'une quelconque des revendications 1 à 11, **caractérisé en ce qu'**il est susceptible d'être obtenu par granulation aqueuse.

13. Comprimé selon l'une quelconque des revendications 1 à 12, **caractérisé en ce qu'**il est préparé par granulation par voie sèche activée par l'humidité (MADG).

14. Processus de fabrication d'une composition pharmaceutique solide, qui est un comprimé selon l'une quelconque des revendications 1 à 13, comprenant une étape de granulation par voie humide de la dronédarone ou de son sel acceptable du point de vue pharmaceutique, de préférence l'hydrochlorure de dronédazrone.

15. Processus de préparation d'un liquide de granulation comprenant la désagglomération et/ou le broyage et/ou la micronisation de l'ingrédient actif, lequel est choisi parmi la dronédarone et ses sels acceptables du point de vue pharmaceutique, de préférence l'hydrochlorure de dronédarone, dans lequel ladite désagglomération et/ou ledit broyage et/ou ladite micronisation de l'ingrédient actif sont mis en oeuvre de façon telle que sa surface spécifique spécifique est supérieure à 1 m²/g et inférieure à 8 m²/g, dans lequel la surface spécifique est déterminée en utilisant le procédé BET, et dans lequel ladite désagglomération et/ou ledit broyage et/ou ladite micronisation de l'ingrédient actif sont optionnellement mis en oeuvre en présence d'au moins un excipient additionnel.
